# EUROPEAN PATENT SPECIFICATION

(11) **EP 1 755 518 B1**
(45) Date of publication and mention of the grant of the patent: **07.08.2013**
(21) Application number: 05752926.5
(22) Date of filing: 24.05.2005
(51) Int. Cl.: A61F 13/472, A61F 13/84

(54) **ABSORBENT ARTICLE HAVING A FIT GUIDE**
SAUGFÄHIGER ARTIKEL MIT EINER PASSFÜHRUNG
ARTICLE ABSORBANT A GUIDE DE POSITIONNEMENT

(30) Priority: 24.05.2004 US 852709; 24.05.2004 US 852629
(43) Date of publication of application: 28.02.2007
(73) Proprietor: The Procter & Gamble Company, Cincinnati, OH 45202 (US)
(72) Inventor: DIGIACOMANTONIO, Marco, I-65125 Pescara (IT); TORO, Evelina, I-66013 Chieti Scalo (IT); CARLUCCI, Giovanni, I-66100 Chieti (IT); BELLUCCI, Remo, I-65010 Spoltore Pescara (IT)
(74) Representative: Briatore, Andrea
(86) International application number: PCT/US2005/018264
(87) International publication number: WO 2005/115296

(56) References cited:
- US-A- 4 605 404
- PATENT ABSTRACTS OF JAPAN vol. 2003, no. 12, 5 December 2003 (2003-12-05) & JP 2003 230592 A (DAIO PAPER CORP), 19 August 2003 (2003-08-19)
- PATENT ABSTRACTS OF JAPAN vol. 2003, no. 06, 3 June 2003 (2003-06-03) & JP 2003 052743 A (DAIO PAPER CORP), 25 February 2003 (2003-02-25)

## Description

### FIELD OF THE INVENTION

The present invention relates to disposable absorbent products, and more particularly to disposable absorbent products and methods for placing and positioning disposable absorbent products intended to be worn in women's undergarments.

### BACKGROUND

Sanitary napkins are used by women principally during their menstrual periods to receive and contain menses and other vaginal discharges to protect their garments from soiling. Sanitary napkins typically have adhesive attachment means to temporarily adhere the device to the crotch region of the user's undergarment, normally her panty.

When placing an absorbent article in an undergarment, it is often critical that the article be positioned correctly with respect to the crotch portion thereof. Improper positioning of the absorbent article can result in bodily discharges coming into contact with the wearer's garments or undergarment, instead of entering the absorbent article. For example, if the absorbent article is place to far toward the front of the undergarment, a rearward portion of the undergarment may not be covered by the absorbent article, resulting in fluid, such as menses, soiling the undergarment. The problem is made worse when the absorbent article is asymmetric, such that it does not give a good indication of proper placement, such as absorbent articles that are narrow in the front and wide in the back, or otherwise are not symmetric about a transverse centerline. Further, if the product has what are commonly referred to as "wings" or "flaps" intended to wrap the edges of the wearer's undergarments in the crotch region and/or affix the article to the undergarment, misplacement of the article can result in poor folding and premature detachment.

Changing the size of the absorbent article can help alleviate the problem of improper placement. However, increasing the size, such as the length, of the article also increases the cost, thereby making this solution commercially unattractive.

Accordingly, there remains an unaddressed need for a feminine hygiene article, such as a sanitary napkin or pantiliner, which is designed to facilitate proper placement and positioning in a user's undergarment.

Additionally, there remains an unaddressed need for a method for properly placing and positioning a feminine hygiene product, such as a sanitary napkin, in a user's undergarment.

Further, there is an unaddressed need for a means for properly placing and positioning an absorbent article in an undergarment when the absorbent article is not symmetric about a longitudinal and/or transverse centerline thereof.

Further, there remains an unaddressed need for a method for properly placing and positioning a feminine hygiene product, such as a sanitary napkin in an undergarment when the feminine hygiene product is not symmetric about a longitudinal and/or transverse centerline thereof.

### SUMMARY OF THE INVENTION

An absorbent article according to the claims having a longitudinal centerline and adapted to be worn in an undergarment having a crotch portion bounded on opposite sides by portions of curved leg openings is disclosed. The absorbent article comprises a main body portion, the main body portion comprising a liquid pervious body-facing surface, a liquid impervious garment facing surface, and an absorbent core positioned between the body-facing surface and the garment-facing surface. The absorbent article comprises at least one indicator marker visible from the body-facing surface, the indicator markers disposed to indicate proper alignment of the absorbent article with respect to undergarment-specific features such as the curved leg openings. A method for correctly placing an absorbent article in the crotch portion of an undergarment having a crotch portion bounded on opposite sides by portions of curved leg openings is also disclosed.

### BRIEF DESCRIPTION OF THE DRAWINGS

FIG. 1 is a partially cut away plan view of an absorbent article of the present invention.
FIG. 2 is a cross-sectional view of the section 2-2 as shown in FIG. 1.
FIG. 3 is a plan view of an absorbent article of the present invention properly positioned in an undergarment.
FIG. 4 is a plan view of another embodiment of an absorbent article of the present invention properly positioned in an undergarment.
FIG. 5 is an enlarged detail view of an indicator marker of the present invention.
FIG. 6 is a partial plan view of a properly aligned absorbent article of the present invention.
FIG. 6A is an enlarged view of a portin of Fig. 6.
FIG. 7 is a partial plan view of an improperly aligned absorbent article of the present invention.
FIGS. 8-21 illustrate various alternative embodiments of sanitary napkins having indicator markers of the present invention.

### DETAILED DESCRIPTION OF THE INVENTION

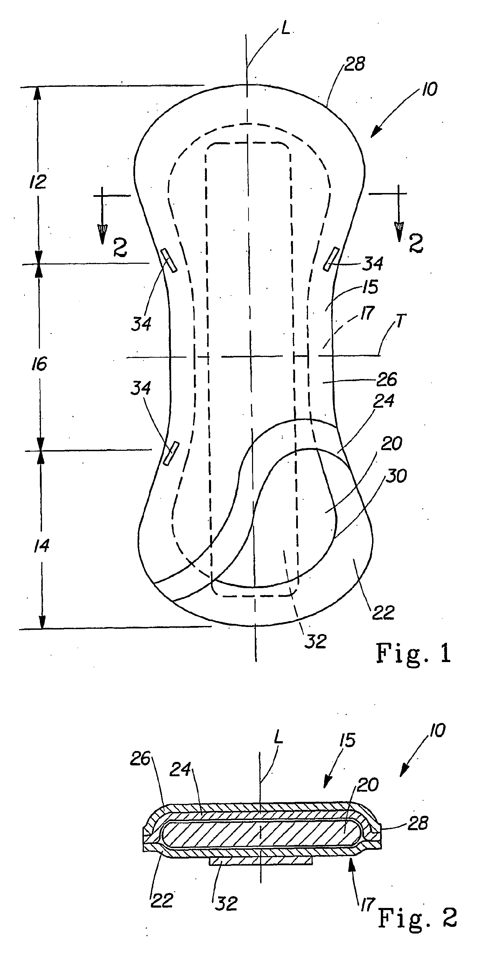

One embodiment of an absorbent article of the present invention, a sanitary napkin **10,** which can be a pantiliner or the like, is shown in partially cut-away plan view in FIG. 1 and in cross section in FIG. 2. A preferred embodiment is a feminine hygiene article, such as a sanitary napkin or pantiliner. While the invention is disclosed in a particularly preferred embodiment of a sanitary napkin, the described invention can also be considered as a pantiliner, and all descriptions below with respect to sanitary napkins can be pantiliners as well, with the difference being one of degree rather than kind. The invention can also be an adult incontinence device, an anal discharge pad, an interlabial pad, or any other absorbent article for which proper placement and positioning in the crotch portion of an undergarment is desirable.

The sanitary napkin **10** has two end regions **12** and **14** and a middle region **16.** The sanitary napkin **10** has a body-facing side **15** that is in contact with the user's body and a garment facing **17** side that is in contact with the inner surface of the user' undergarment. In general, each component layer of the sanitary napkin **10** can be said to have a body-facing side and a garment-facing side, the sides being determined by their orientation relative to the use of the article. Sanitary napkin **10** has a longitudinal centerline **L** and a transverse centerline **T** that are perpendicular to one another in the plane of the sanitary napkin when in a flat out configuration, as shown in FIG. 1. In one embodiment sanitary napkin is generally symmetric about both longitudinal axis **L** and transverse axis **T.** However, in other embodiments, sanitary napkin **10** may be asymmetric about either axis.

While the sanitary napkin **10** may have any shape known in the art, one preferred shape, shown in FIG. 1, is generally "hourglass" shaped, tapering inwardly from a relatively greater transverse width in a portion of one of the end regions to a relatively smaller transverse width at the middle region. Transverse width is generally defined as the dimension across the article, measured parallel to the transverse centerline **T.** As discussed more fully below, sanitary napkins can also be provided with lateral extensions known commonly in the art as "flaps" or "wings" (not shown in FIG. 1) intended to fold over and cover the panty elastics in the crotch region of the user's undergarment.

Sanitary napkin **10** can have an absorbent core **20** to absorb and store bodily fluids discharged during use. Absorbent core **20** can be formed from any of the materials well known to those of ordinary skill in the art. Examples of such materials include multiple plies of creped cellulose wadding, fluffed cellulose fibers, wood pulp fibers also known as airfelt, textile fibers, a blend of fibers, a mass or batt of fibers, a web of polymeric fibers, and a blend of polymeric fibers.

In one embodiment absorbent core **20** can be relatively thin, less than about 2 mm in thickness, preferably less than about 1 mm, and more preferably less than about 0.5 mm in thickness. Thickness can be measured by any means known in the art for doing so. The absorbent core can comprise absorbent gelling materials (AGM), including AGM fibers, as is known in the art.

Absorbent core **20** can be formed or cut to a shape, the outer edges of which define a core periphery **30**. The shape of absorbent core **20** can be generally rectangular, circular, oval, elliptical, or the like. Absorbent core **20** can be generally centered with respect to the longitudinal centerline **L** and transverse centerline **T**.

To prevent absorbed bodily exudates from contacting the wearer's garments, sanitary napkin **10** can have a liquid impermeable backsheet **22**. To provide a degree of softness and vapor permeability for the garment-facing side of sanitary napkin **10**, backsheet **22** can be a vapor permeable outer layer on the garment-facing side of the sanitary napkin **20**. The backsheet **22** can be formed from any vapor permeable material known in the art. Backsheet **22** can comprise a microporous film, an apertured formed film, or other polymer film that is vapor permeable, or rendered to be vapor permeable, as is known in the art. One suitable material is a soft, smooth, compliant, vapor pervious material, such as a nonwoven web that is hydrophobic or rendered hydrophobic to be substantially liquid impermeable. A nonwoven web provides for softness and conformability for comfort, and is low noise producing so that movement does not cause unwanted sound.

To provide for softness next to the body, sanitary napkin **10** can have a body-facing layer, referred to herein as topsheet **26**. Topsheet **26** can be formed from any soft, smooth, compliant, porous material which is comfortable against human skin and through which fluids such as vaginal discharges can pass. Topsheet **26** can comprise fibrous nonwoven webs and can comprise fibers as are known in the art, including bicomponent and shaped fibers. Topsheet **26** can also be a liquid permeable polymer film, such as an apertured film, or an apertured formed film as is known on sanitary napkins such as ALWAYS® brand sanitary napkins.

At least one, and preferably both, of topsheet **26** and backsheet **22** define a shape, the edge of which defines an outer periphery **28** of the sanitary napkin. In a preferred embodiment, both topsheet **26** and backsheet **22** define the sanitary napkin outer periphery **28**. The two layers can be die cut, as is known in the art, for example, after combining all the components into the structure of the sanitary napkin as described herein.

Interposed between the absorbent core **20** and topsheet **26** can be a fluid permeable secondary topsheet **24**. Secondary topsheet **24** can aid in rapid acquisition and/or distribution of fluid and is preferably in fluid communication with the absorbent core **20**. In one embodiment, the secondary topsheet **24** does not completely cover the absorbent core **20,** but it can extend to core periphery **30**.

In one embodiment, absorbent core **20** does not extend laterally outward to the same extent as either topsheet **26** or backsheet **22,** but the sanitary napkin outer periphery **28** can be substantially larger than the core outer periphery **30**. In this manner, the region of sanitary napkin **10** between the core periphery **30** and the sanitary napkin outer periphery **28** can define a breathable zone **32** that permits vapors to go through portions of the sanitary napkin, thereby escaping and providing for dryer comfort when worn. The breathable zone and sanitary napkin having a breathable zone can be according to the teachings of U.S. Pat. Appl. No. 10/790,418, filed March 1,2004, published as US 2005/0192549A1.

All the components can be adhered together with adhesives, including hot melt adhesives, as is known in the art. The adhesive can be Findlay H2128 UN and Savare' PM 17 can be applied using Dynafiber HTW system.

As is typical for sanitary napkins and the like, the sanitary napkin **10** of the present invention can have panty fastening adhesive **32** disposed on the garment-facing side **17** of backsheet **22**. Panty fastening adhesive **32** can be any of known adhesives used in the art for this purpose, and can be covered prior to use by a release paper, as is well known in the art.

The above disclosure is meant to give a general description of the basic parts of feminine hygiene articles such as sanitary napkins and the like as they are known in the art. The description is not intended to be limiting. Any and all of various known elements, features and processes of known sanitary napkins, pantiliners, incontinence pads, and the like can be incorporated in an absorbent article of the present invention as desired or needed for particular use benefits. Now, with respect to the remaining disclosure, the novel features and benefits of the present invention will be described.

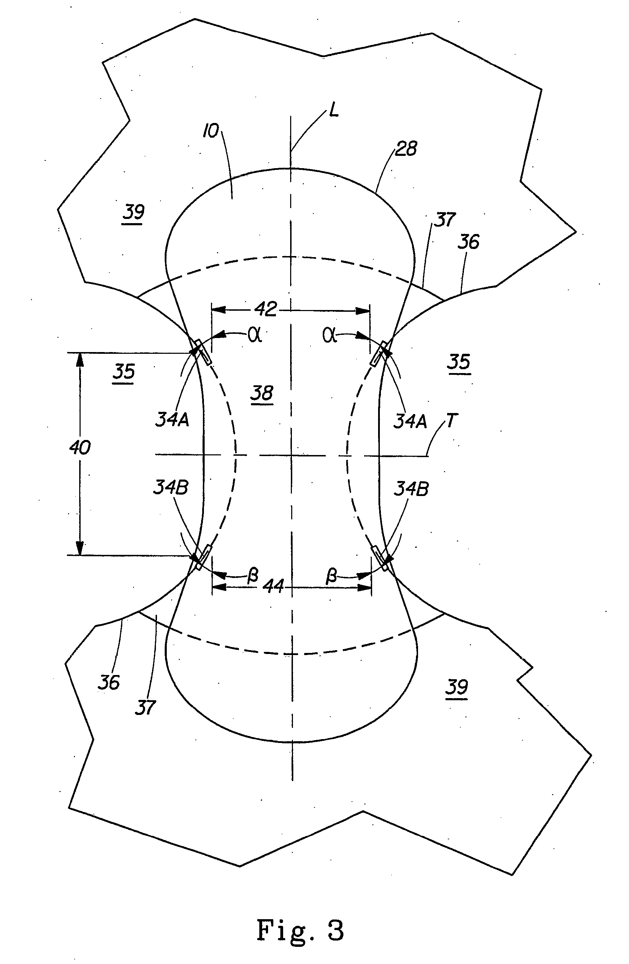

As shown in FIG. 1, an absorbent article of the present invention has at least one indicator marker **34** visible from the body-facing surface **15**. The indicator markers **34** provide an indication for proper fit when placing and positioning the sanitary napkin **10** in an undergarment, such as the panty of the wearer. Indicator markers **34** can comprise printed indicia, such as ink-jet printed lines or line segments, embossed ridges or bumps, folds, pleats, or any other means known in the art for providing visible indications that serve the function of being indicator markers **34**, which is to aid the user in determining proper placement of the article in an undergarment. Specifically, as shown in FIG. 3, indicator markers **34** can be disposed so as to indicate proper placement of the absorbent article with respect to undergarment-specific features such as an undergarment **39**. For example, the crotch portion **38** of the undergarment **39** can have seams **37** associated therewith, and is bounded on opposite sides by elasticized leg elastics **36** of the curved leg openings **35**.

Indicator markers **34** can be disposed to be visible from the body-facing surface **15** such that an indication is made to the user as to the proper positioning in the undergarment. Undergarments typically have undergarment-specific features such as sewn seams, hemmed edges, elasticized leg openings, additional crotch panel material, and other visibly-distinct portions that can be used for alignment with indicator markers **34**. Therefore, in some embodiments of the present invention, indicator markers **34** can be disposed so as to indicate alignment with a sewn seam, such as seam **37** of a crotch panel of the panty of the wearer. A crotch panel can be, for example, an additional piece of cotton material sewn into the crotch portion **38** of a panty-style undergarment **39**. However, in a preferred embodiment, indicator markers **34** are disposed to indicate alignment and fit of a sanitary napkin with respect to the leg elastics **36** of the undergarment, as discussed more fully below.

FIG. 3 shows a sanitary napkin **10** outer periphery **28** as seen when properly positioned in the crotch portion **38** of a panty **39**. Sanitary napkin **10** shown in FIG. 3 is an example of an article of the present invention not having flaps intended to wrap the edges of the leg openings of the undergarment. As shown in FIG. 3, portions of the panty **39** defining leg openings **35**, such as leg elastics **36**, can be positioned under a portion of sanitary napkin **10** when the sanitary napkin **10** is properly placed in the crotch region **38** of the panty. To aid in placing and positioning the sanitary napkin **10** properly, in one embodiment indicator markers **34** can be disposed so as to indicate proper alignment of the absorbent article **10** with respect to the curvature of leg openings **35** of the undergarment **39**.

As shown in FIG. 3, indicator markers **34** can be placed so as to give the user a visual indication of proper placement of the article in the undergarment. Because the actual curvature of leg elastics in panty leg openings can be asymmetric about the transverse axis **T** of the article **10**, in one embodiment indicator markers **34** can differ in size and orientation based on their location with respect to the panty leg opening. For example, in FIG. 3, indicator markers **34A** can be associated with the forward or "front" portion of the leg openings (that is, the portion of the leg openings more toward the front of the wearer). Likewise, indicator markers **34B** can be associated with the rearward or "back" portion of the leg openings.

Undergarment styles can differ greatly, and the style of undergarment, such as panties, can have an impact on the curvature of the leg openings **35** bounding the crotch portion **38** of the undergarment. For this reason, absorbent articles, such as sanitary napkin **10**, can be designed for various panty sizes by varying the disposition of indicator markers **34** in a lengthwise direction as indicated by dimension **40** on FIG. 3, and in a widthwise direction, as indicated by dimensions **42** and **44** on FIG. 3. Dimensions **42** and **44** can be different or substantially the same. Dimensions **42** and **44** can be measured in any convenient way, such as from "inside-to-inside" (least dimension between marks, measured laterally parallel to transverse centerline **T**) or "outside to outside" (greatest dimension including marks measured laterally parallel to transverse centerline **T**) or from "center to center" as is most appropriate for the type of indicator marker. In one embodiment, a minimum dimension measured inside-to-inside can be 20 mm, and a maximum dimension measured inside-to-inside can be 85 mm, and the inside-to-inside dimension can be any measurement of increments of 1 mm between the minimum and maximum. For "thong" type panties, for example, it may be desirable to have indicator markers **34B** separated by the minimum inside-to-inside dimension of about 20 mm.

In one embodiment indicator markers **34** are generally elongated in shape, and can be generally linear or have a slight curvature. For example, as shown in FIG. 3, indicator markers **34** can be generally linear-shaped rectangular marks or line segments. Indicator marks **34** can be made by printing, stamping, embossing, folding or any other known process that makes a visual, or even tactile, impression that indicates proper alignment for the user. In the case of generally linear marks, such marks can make an angle with respect to the longitudinal centerline **L**. The angle can be the same for all indicator marks, or different angles can be used for the forward indicator marks **34A** and rearward indicator marks **34B.** As shown in FIG. 3, forward indicator markers **34A** can be oriented at an angle α, and rearward indicator markers **34B** can be oriented at an angle β from the longitudinal centerline **L**. As used herein all angle measurements are taken as being positive and increasing away from a line parallel to the longitudinal centerline **L** and having a vertex oriented nearer the transverse centerline **T.** Angle α can be oriented in a range in 1 degree intervals from about 5 degrees to about 45 degrees. Likewise, angle β can be oriented in a range in 1 degree intervals from about 5 degrees to about 45 degrees.

The indicator markers **34** of the present invention are particularly beneficial when incorporated on an absorbent article that is asymmetric about either of the transverse centerline **T** or longitudinal centerline **L,** and/or an absorbent article that incorporates flaps for folding about the leg elastics **36** of the undergarment **39.**

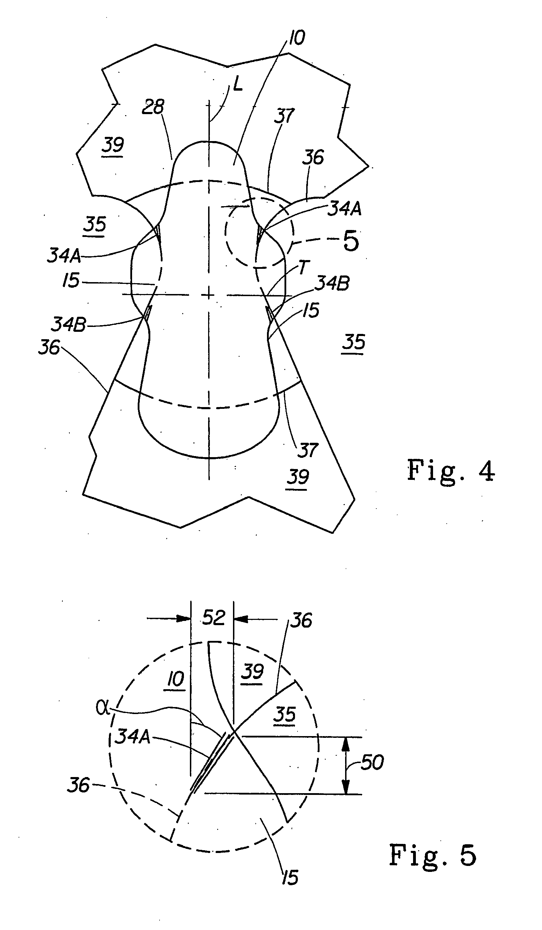

FIG. 4 shows a sanitary napkin **10** having flaps **15** that are intended to fold over and wrap the leg elastics (flaps **15** in FIG. 4 are shown in an unfolded, flat condition). Flaps **15** can have attachment means (not shown) to affix the sanitary napkin to the underside of the undergarment, as is known in the art. Any of various attachment means known in the art can be used with the present invention, including pressure sensitive adhesive means, in which case release strips can be incorporated as well. It is known to make a line of weakness or a flexible zone to facilitate folding of flaps on sanitary napkins. However, such fold lines are not to be confused with the indicator markers **34** of the present invention. Whereas fold lines (if noticeable at all by the consumer) are simply made to permit folding around a wide range of panty sizes and styles, they are not necessarily sufficiently shaped, placed, or otherwise designed to be fit guides for proper placement and positioning. Therefore, since they permit incorrect placement and positioning of the sanitary napkin, they are not indicator markers **34**. In some embodiments, indicator markers **34** of the present invention can be in addition to and distinguished over flap fold lines. That is, indicator markers **34** can be visually or tactilely distinct over any fold lines for flaps.

FIG. 4 also illustrates a sanitary napkin **10** that is asymmetric about the transverse centerline **T**. One way of describing such a sanitary napkin **10** is to say that one of the end regions **12** or **14** is longer in a dimension parallel to the longitudinal centerline **L** than the other. Such a sanitary napkin, when properly placed in an undergarment can result in more of the absorbent capacity toward the rear of the user's undergarment for better protection from soiling. In general, a central region **16** is designed to be placed in the narrowest portion of the crotch region of a user's undergarment. However, asymmetric designs present a problem to the user. Because the pad is not designed for the transverse centerline **T** to be in the center of the crotch region **38**, the user can very easily mis-position the article in her undergarment. In general, it has been found that for asymmetric sanitary napkins of the type illustrated in FIG. 4, users typically position the article too far forward in their undergarment. This is believed due to force of habit since users typically place absorbent articles such that the pad is generally centered with respect to the central portion of the crotch of the undergarment. Such misplacement has been shown to have a direct correlation to reduced fit, reduced comfort, and increased soiling conditions, such as spotting of the user's undergarments.

The indicator markers **34** of the present invention aid in proper placement by giving the user a visual signal of proper positioning with respect to the crotch portion of the undergarment. Especially for asymmetrical sanitary napkins, such a visual signal has been found to greatly increase the frequency of proper placement among sanitary pad users. As shown in FIG. 4, and more clearly in the enlarged portion shown in FIG. 5, indicator markers **34** can be on the sanitary napkin **10** to indicate proper positioning with respect to the curvature of leg elastics **36**. Each indicator marker **34** can comprise a plurality of linear indicators, such as the three line segments shown in FIGS. 4 and 5, and the plurality of linear indicators can be generally parallel to one another, or they can be non-parallel, as shown in FIG. 5. When individual marks of an indicator marker **34** are non-parallel, each mark can be oriented at an angle that is greater in proportion to an increased distance measured from the longitudinal centerline **L.** In such a case, the angle α for the plurality of marks can be considered to be the average of the angles for a given plurality of indicator marks making up one indicator marker **34.**

As shown in FIG. 5, indicator marker **34A** (and, in general indicator markers **34**) can be a single mark (or embossment, fold, pleat, and the like) or a plurality of individual marks. In either case, indicator marker **34** can be measured and quantified in at least two ways. As shown in FIG. 5, indicator marker **34** can have a length dimension, indicated as **50** and a width dimension indicated as **52.** These dimensions, measured to the extremities of indicator marker **34** (including individual marks, if necessary) parallel to the longitudinal centerline **L** and the transverse centerline **T,** respectively, define an angle α (or β for rearward indicator markers **34B**) and a ratio **R1** of length **50**/width **52** which is defined as 1/tan α. For non-parallel, "fanned-out" marks (or pleats, etc.), as shown in FIG. 5, angle α can correspond to an intermediate angle, such as an average angle of all the marks. In one embodiment, the ratio **R1** can be greater than 1, and can be greater than 2, 3, 4, 5, 10, 15, or more. In general, the angle α necessary for proper fit guidance will be determined by the size of the article, its symmetry about either of the longitudinal or transverse axes, and the type and size of undergarment for which it is designed. It has been found that an angle α (or β for rearward indicator markers **34B**) of between about 10 degrees to about 30 degrees, variable in 1 degree increments, is sufficient. In one embodiment having "fanned-out" marks (or pleats, etc.), as shown in FIG. 5, angles α or β can vary for each individual mark from between about 10 degrees to about 30 degrees.

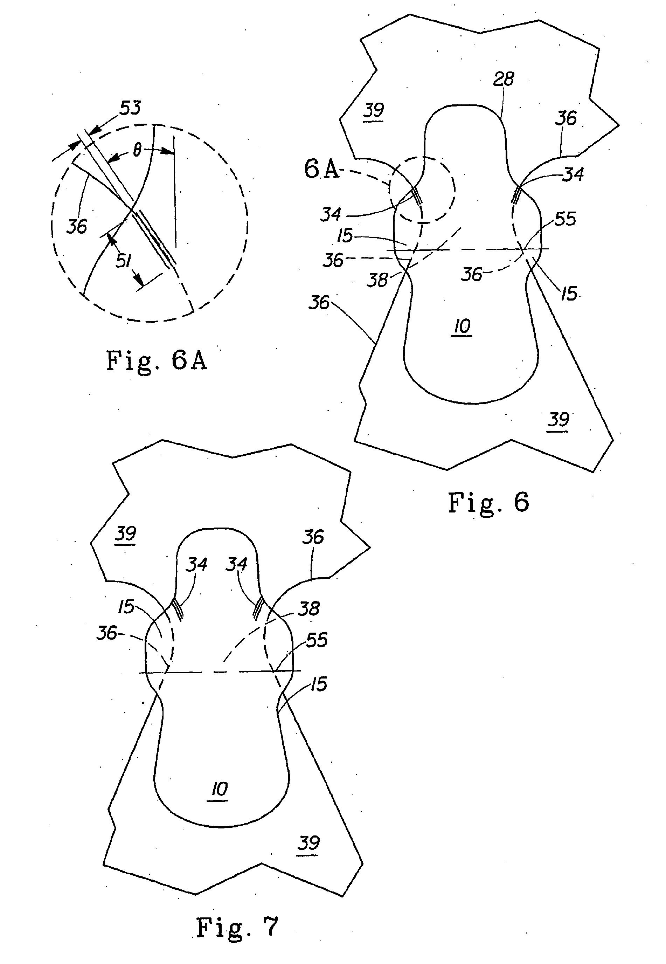

A second way of quantifying the configuration of indicator markers **34** is shown with respect to FIGS. 6 and 6A. As shown, an effective length dimension, indicated as **51** and an effective width dimension indicated as 53. Measured in this manner, a second ratio **R2** of dimension **51**/dimension **53** can be defined. Defining ratio **R2** permits the indicator marker **34** to be specified by ratio **R2** and an independently-determined angle Θ. In general, ratio **R2** can be greater than 1, and can be greater than 2, 3, 4, or 5. Angle Θ can be at least about 10 degrees measured relative to a line parallel to longitudinal centerline L, and the first laterally-outboard mark (or pleat) of indicator marker **34,** as shown in FIG. 6A. Angle Θ can be at least about 15 degrees, at least about 20 degrees, at least about 25 degrees.

In general, it has been found that indicator markers **34** having a relatively high ratio **R1** or **R2 of** about 2 to 5, and a relatively low angle α or β or Θ of between about 10 degrees to about 30 degrees, provides for an absorbent article having indicator markers **34** suitable for a wide range of panty sizes. If a plurality of angled marks is used, such as shown in FIGS. 5 and 6, it is desirable that the minimum and maximum angles (α or β or Θ) fall within the range of about 15~30 degrees for forward indicator markers **34A,** and in the range of about 10 -20 degrees for rearward indicator markers **34B.** In general, the placement of indicator markers **34,** including with respect to a length dimension **40** or width dimensions **42** or **44,** can be varied to correlate to predetermined panty sizes.

As shown in FIGS. 6 and 7, indicator markers **34** can serve as a fit guide to properly position a sanitary napkin **10** in an undergarment by indicating proper alignment with respect to leg elastics **36.** As shown in FIG. 6, a properly positioned sanitary napkin **10** aligns indicator markers **34** with leg elastics **36** where the leg elastics **36** intersect sanitary napkin periphery **28.** In this manner, by visual signal, the user can properly position the sanitary napkin **1**0 with respect to the crotch portion **38** of the undergarment **39,** for example, a lateral section 55 of the crotch portion **38** of the undergarment **39.** If sanitary napkin **10 is** improperly positioned, for example positioned too far forward as shown in FIG. 7, indicator markers **34** no longer align with leg elastics **36.** The user, noticing the mis-alignment, can then re-align the sanitary napkin if necessary. By having indicator markers 3**6** comprised of a plurality of marks or folds, a range of proper placement can be provided for, thereby permitting a sanitary napkin to fit a range of undergarment sizes and types.

As indicated in FIGS. 6 and 7 as well, it is not necessary that sanitary napkin **10** comprise both forward and rearward indicator markers **34A** and **34B.** In practice, only forward or rearward indicator markers are necessary. In fact, a workable sanitary napkin need only have one indicator marker **34,** for example one of the left or right of either forward or rearward indicator markers **34A** and **34B.** However, having more than one mark is believed to make positioning easier for the user.

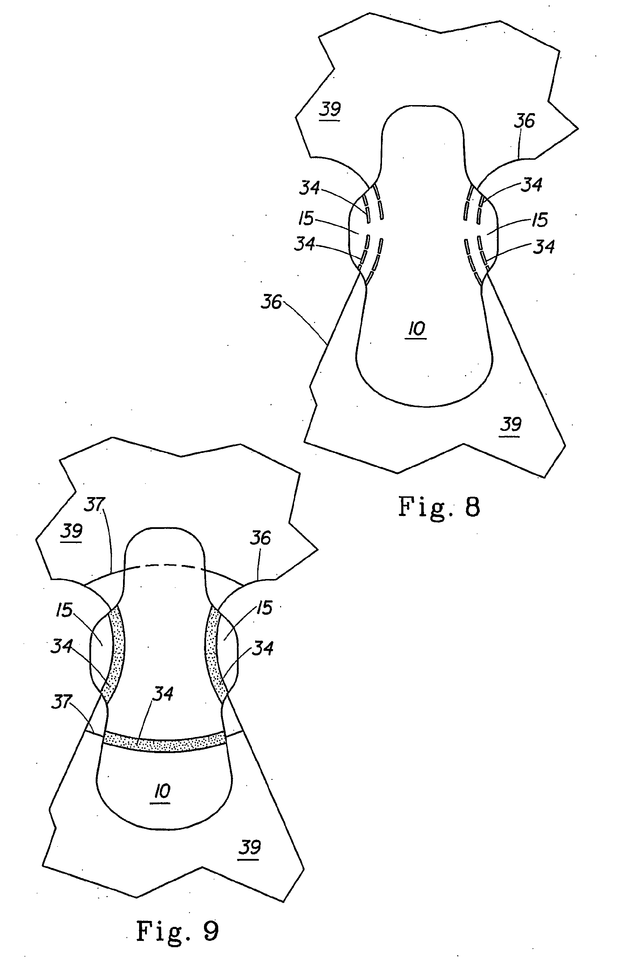

As shown in FIGS. 8-11, indicator markers **34** can be printed on a surface visible from the body-facing surface of sanitary napkin **10.** The indicator markers **34** can be printed on a surface below the topsheet as long as it is visible to the user during placement and positioning of the article in the undergarment. Therefore, indicator markers **34** can be printed, or otherwise disposed on, secondary topsheets, surge layers, acquisition layers, absorbent cores, and the like. Indicator markers **34** can be configured as lines, line segments, curved lines, bands, arrows, words, pictures, or any other printed indicia having a purpose of providing a fit guide indication. Again, the visual indicia need not be printed on the body-contacting surface, but need only be visible from the body-contacting surface such that the user can see the indicator marks **34** as she places the sanitary napkin **10** in her undergarment **39.**

As shown in FIGS. 8 and 9, indicator markers **34** can provide an indication of where the undergarment elastic should go by giving a visual representation of where the elastic should be underneath the article. FIG. 9 shows an embodiment in which an indicator marker **34** is disposed to overlie a seam **37** of the undergarment, such as a seam of a sewn-in crotch panel. Indicator marker **34** overlying seam **37** can be used alone, or in combination with other indicator markers **34** as shown in FIG. 9.

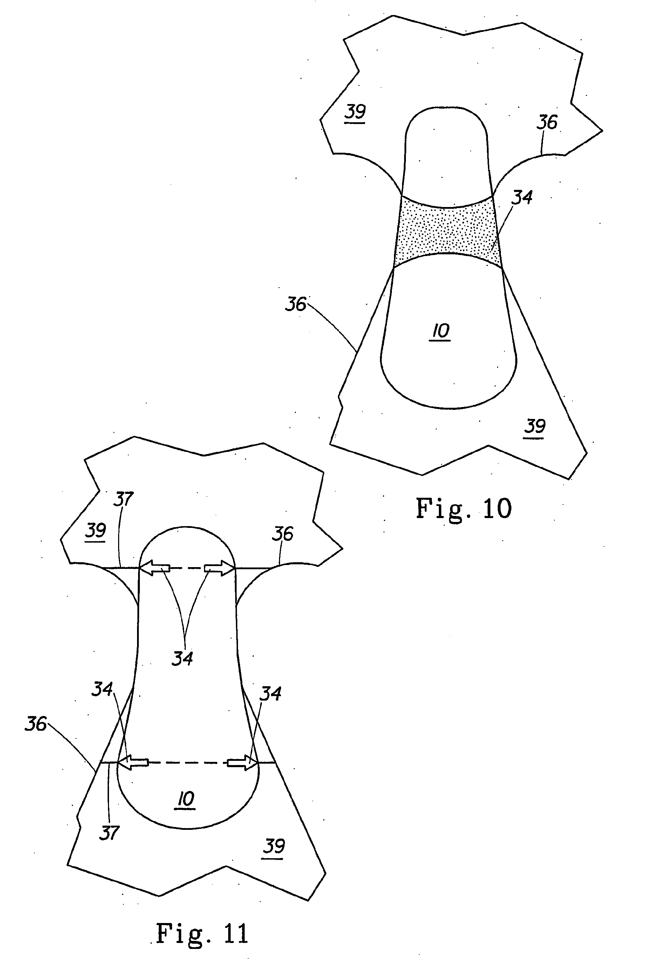

FIG. 10 shows an embodiment in which one continuous indicator marker **34** can provide an indication at all four locations that leg elastics **36** go under the sanitary napkin **10,** resulting in the benefit of proper fit. The indicator marker **34** of FIG. 10 can be a solid colored band that extends across the entire article, including the flaps, if utilized. Alternatively, indicator marker **34** of FIG. 10 can be a shaded, striped, stippled, or other noncontiguous band that gives the impression of being a continuous colored band. In one embodiment, the "comers" of the band as shown in FIG. 10 can line up with the edges of the article which cross over the leg elastics 36 of undergarment **39.**

FIG. 11 shows an embodiment in which printed arrows indicate the points at which seams **37** should cross under the article **10.** In one embodiment, multiple indicator markers **36** can be utilized and appropriately marked to convey fit for various sized undergarments.

Rather than utilize printed-on marks for indicator markers **34,** the article of the present invention can utilize other visual or tactile indicia. For example, indicator markers **34** can be differentially extensible zones on flaps **15** as taught in WO 97/12577, issued 10 April 1997 to Lash et al.; or WO 96/12461, issued 19 October 1995 to Gellich et al. Differentially extensible zones are formed by mechanically straining portions of the sanitary napkin to permanently stretch and deform ridges and valleys. Such straining can be by the method commonly referred to as ring-rolling, or strained areas can be local strainable networks made by what is referred to in the art as SELF'ing. However, these embodiments show extensible zones having configurations that are not suited for use as indicator markers **34** of the present invention. In general, the deformed regions suitable for extensible zones extend to far laterally-outward into the wing area, thereby not providing for reliable fit guides.

Even though deformed regions modified to be indicator markers **34** can be extensible zones that aid in wrapping and conforming the wing about the leg elastics in the crotch region of an undergarment, it is clear that the indicator markers **34** need not perform the function of stretch or extension to aid in wrapping the flaps to better conform to the undergarment. They simply need to provide a visual indication of proper fit, such as having the ridges being properly angled and of a sufficient length to align with the panty elastics when worn in an undergarment of the appropriate size.

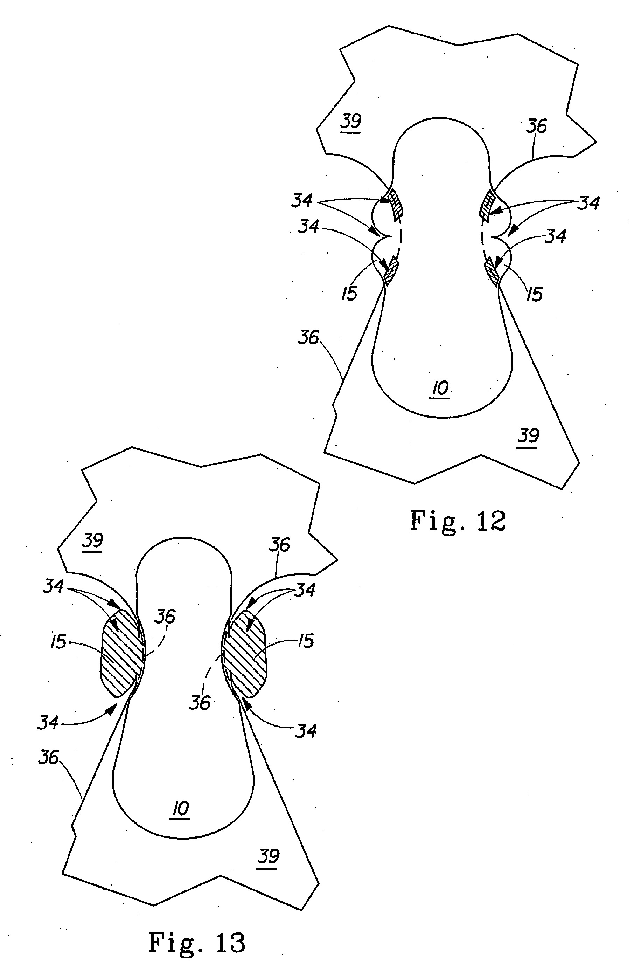

Aside from printed marks, other indicator markers **34** can be utilized. For example, as shown in FIGS. 12 and 13, notches or indentations can be utilized in conjunction with other indicator markers **34**. In FIG. 12, notches in flaps **15** can indicate by "pointing," for example, to where the center of the crotch portion **38** of undergarment **39** should be with respect to sanitary napkin **10**. Such a pointer can reinforce in the users mind the correct placement as indicated by, for example, printed indicator marks **34** to mark the cross-over point with the panty elastics **36**. In FIG. 13, notches at the base of flaps **15** can function together with a printed-on indication in the wings **15** as indicator markers **34** by giving the user an indication of where leg elastics **36** should be disposed with respect to sanitary napkin **10**.

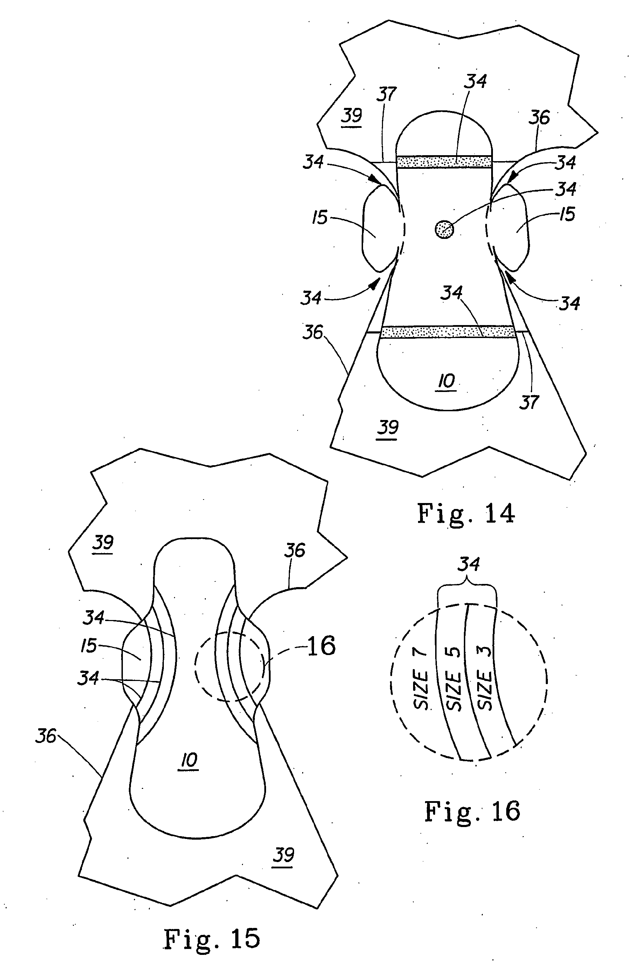

Other embodiments, for example, a combination of a centrally-printed mark, seam-indicator marks, and notches, as shown in FIG. 14 can be utilized. As shown in FIG. 14, a centrally-placed marking, such as a shaded circular shape, can act as an indicator marker 34, and can be utilized alone or together with other indicator markers, as shown.

FIG. 15 shows an embodiment using printed indicia in the form of lines, words and numbers. In this embodiment a series of lines of indicator markers **34** can be provided that correspond to typical shapes of leg openings and crotch widths for various sizes of undergarments. Even if the user's undergarment does not match exactly to the indicator markers **34** provided, such a configuration provides a visual signal of the central region of the absorbent pad anyway, and can be used to place such a central region properly with respect to the crotch portion **38** of an undergarment **39**.

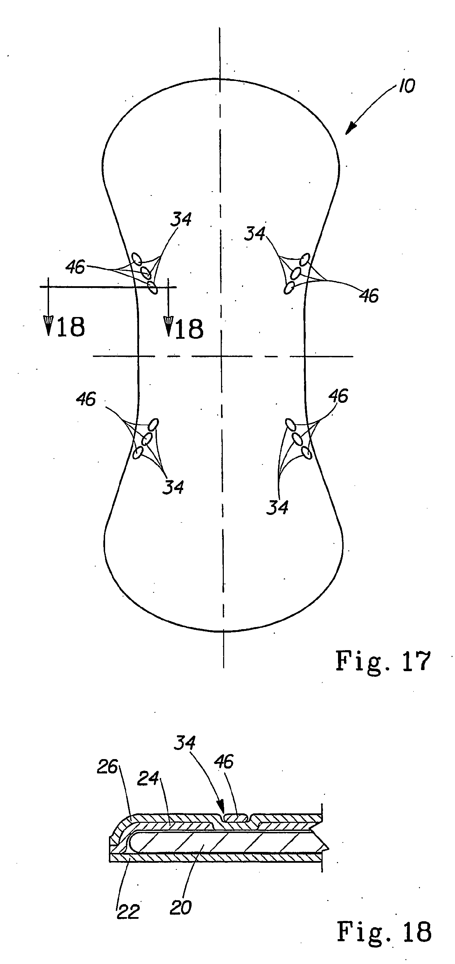

FIGS. 17 and 18 illustrate another embodiment of the present invention combining embossing and printing or other methods known in the art for imparting color, to make indicator markers **34**. As shown in FIG. 17, sanitary napkin **10** can comprise indicator markers **34** comprising a plurality of embossments that together form a generally linear indicator mark **34**. As shown in FIG. 18, one or more of the embossed impressions, preferably all the embossed depressions can have therein a visible color **46**. The visible color can be printed in registration with the embossments, or printed at the same time as the embossments are made. The color **46** can be due to printing inks or colored adhesive. The color **46** can also be under the topsheet, even on a separate layer (neither shown in FIG. 18), such that upon embossing, color **46** shows through the embossed portion, thereby giving the appearance of having been printed in registration with the embossed indicator marker **34**.

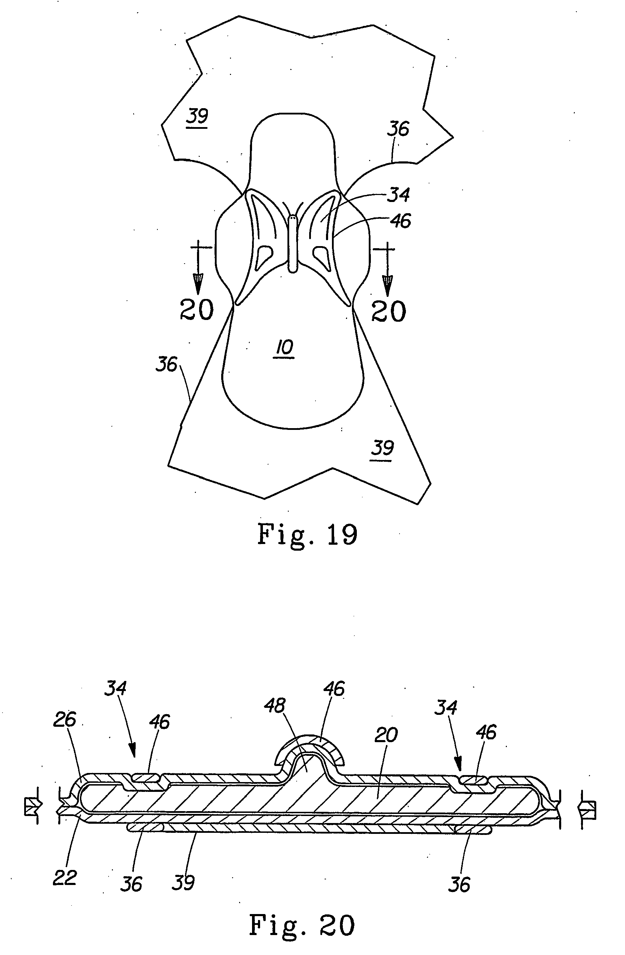

FIGS. 19 and 20 illustrate a further embodiment in which printing and embossment are utilized together. As shown in FIG 19, indicator marker **34** can be a recognizable image, such as a butterfly, the image having components that indicate fit, such as the tips of the butterfly wings that coincide with the intersection of the sanitary napkin **10** and leg elastics **36**. As shown in FIG. 20, certain features of the image can be colored by printing visible colored portions **46** in a printed image that can be enhanced by embossing (as in the wings in the illustrated embodiment), or by debossing (i.e., creating raised portions, as in the body of the butterfly in the illustrated embodiment). As discussed with respect to FIGS. 17 and 18, the colored portions **46**, made by printing or other methods known in the art for imparting color, can be registered with embossed or debossed regions to give a synergistic image of indicator markers **34** useful as a fit guide in a disposable absorbent article.

The embossed and debossed portions of the fit guide can provide a tactile impression that can be used as a fit guide as well. For example, the user can gently touch the edges of the sanitary napkin and follow the line of an embossment, for example, while visually comprehending that the leg elastic **36** follows the felt path of her finger. In a separate but related benefit, the debossed, or raised portion of an image such as the butterfly in FIG. 19 can aid in assessing proper fit by providing to the user a tactilely evident positioning guide. The raised portion can be made, for example, to fit over or in the labial region of the wearer's anatomy, and misplacement can be immediately detected upon pulling the undergarment into place.

In another embodiment, a fit guide can be achieved by having indicator markers that are only tactilely sensed, i.e., not visibly sensed. Such a tactile fit guide can serve as an indicator marker **34** by means of a change in surface smoothness, a change in the coefficient of friction, or other tactilely-sensed change in material properties. In general, the change in material properties can correspond in location to the visible indicator markers **34** as disclosed herein.

In another embodiment, fit guide can be achieved by having indicator markers **34** that indicate by means of variations in the bending stiffness of flaps **15** on sanitary napkin **10**. In this embodiment, the regions indicated in previously-disclosed embodiments corresponding to indicator markers **34** can have a relatively lower resistance to bending about the leg elastics **36** of the undergarment **39**. Upon attempting to fold the flaps over the crotch portion of the undergarment, the user can feel resistance to bending when the sanitary napkin is not positioned correctly within the undergarment. For example, the flap **15** can be relatively stiff, such that it resists bending except about a line of juncture with sanitary napkin **10** corresponding to path of the leg elastics **36** of the undergarment **39**.

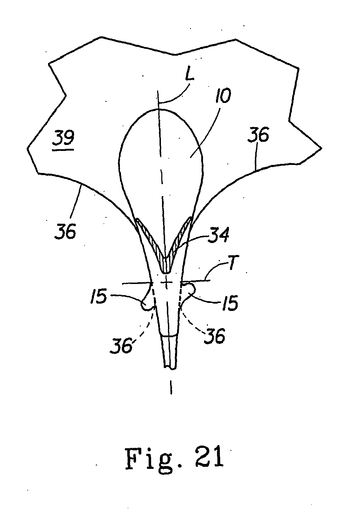

An example of a sanitary napkin (or pantiliner) that is neither symmetric about the longitudinal axis nor the lateral axis is shown in FIG. **21**. FIG. 21 shows a sanitary napkin **10** designed for, and being worn in the crotch portion of a so-called thong, or string panty. The very narrow crotch width requires flaps **15**, if used to be offset with respect to one another so as to avoid overlapping. As shown, an indicator mark **34**, in this case a printed, colored, stylized "V" can indicate proper placement and positioning with respect to the forward leg elastics **36**.

Many variations on the above-described indicator markers are contemplated. For example, indicator markers can comprise sensory perception agents, such as menthol lactate in a sufficient amount so as to give the user a feeling of cooling refreshment when the sanitary napkin is properly placed and worn.. The indicator markers can be made such that, rather than printing with ink, a material is modified so as to have a different reflective index, or even be transparent, in the region intended to be a indicator marker. Likewise, instead of ink, color can be added by adding colored material in appropriate places, the colored material being additional film, nonwovens, or adhesives, including glue and hot melt adhesives. Such color can be added in or on any component of the sanitary napkin, as long as they are visible to the user when she is positioning the sanitary napkin in her undergarment.

In one embodiment a disposable absorbent article of the present invention can be packaged either singly or in a package with other like articles. The package can be labeled as to the size of undergarment(s) the articles are intended to fit, as well as instructions for use, i.e., a method of properly placing and positioning the absorbent article into the undergarment.

A sanitary napkin of the present invention can be used by following the method herein described. First, if there is a choice of sanitary napkins differentiated by intended undergarment size, the user can choose the sanitary napkin for the size undergarment she wears.

It is preferred that the user place the sanitary napkin into her undergarment while the undergarment is being worn, but pulled down about her legs such that the crotch portion thereof is visible and accessible. The user can then remove one sanitary napkin from the packaging, including any individual wrappers, if any. If the sanitary napkin is provided with pressure sensitive adhesive attachment means, the user can remove any backing strips, release paper, or other covering to expose the adhesive.

Once the sanitary napkin is unwrapped, unfolded, or otherwise prepared for placement, the user can observe (or feel, if tactile) the indicator mark(s) provided thereon and visible (or felt) from the body-facing surface thereof, and place the sanitary napkin in her undergarment while spreading the crotch portion thereof with her legs and visibly lining up the indicator mark(s) with the crotch portion of the undergarment, for example, by lining up the indicator mark(s) with at least one leg elastic, and preferably at least two leg elastics at the location where the leg elastics meet or go under the sanitary napkin. Since the leg elastics are stretched while the user is performing the placement, the indicator markers of the sanitary napkin can be designed for the orientation of the leg elastics during placement, rather than during wear.

Placement can be achieved by known methods, such as by exposing (such as by removing a release paper) pressure sensitive adhesive on the garment-facing side of the sanitary napkin, and pressing the sanitary napkin into the crotch portion of her undergarment.

After placement, the user can check for proper positioning, and, if necessary, remove and replace the sanitary napkin for better alignment of the indicator mark(s) with the crotch portion of the undergarment. This step can be repeated as necessary.

If the sanitary napkin is provided with flaps, the user can then fold the flaps down and under the crotch portion of the undergarment, and, if provided for, affix the flaps to the undergarment by means provided, such as by adhesive attachment means.

The user can then pull up her undergarment, assured that the sanitary napkin is properly placed for optimal functioning in maximizing absorbency while minimizing garment soiling.

To aid the user in properly positioning absorbent articles such as a sanitary napkins having indicator markers, that is, to aid the user in using the indicator markers as a fit guide, the sanitary napkin can be provided with instructions for use. Instructions for use can be provided on or in the packaging in which the sanitary napkin is sold, on related advertising or display media, or on the sanitary napkin itself. The instructions can be printed on packaging, such as on an outside surface thereof, or on a separate paper placed inside the packaging. A package can comprise a plurality of absorbent articles, and each absorbent article can be individually wrapped or packaged, as is commonly known in the art. Instructions for use can include indicia such as text and pictorial diagrams. The printed instructions can include instructions for choosing an absorbent article of the present invention based on the size of the user's undergarment.

## Claims

1. A feminine hygiene article (10) having a longitudinal centerline (L) and adapted to be worn in an undergarment (39) having a crotch portion (38) bounded on opposite sides by portions of curved leg openings (35), said feminine hygiene article comprising a main body portion, said main body portion comprising a liquid pervious body-facing surface (15), a liquid impervious garment-facing surface (17), and an absorbent core (20) positioned between said body-facing surface and said garment-facing surface, said feminine hygiene article **characterized by**:
a. at least one indicator marker (34) visible from said body-facing surface, said indicator marker disposed to indicate proper alignment of said feminine hygiene article (10) with respect to said curved leg openings (35), wherein said at least one indicator marker is printed marks.

2. The feminine hygiene article of Claim 1, wherein said feminine hygiene article comprises a transverse centerline (T) perpendicular to said longitudinal centerline (L), and wherein said feminine hygiene article is not symmetric about at least one of said longitudinal (L) or said transverse centerlines (T).

3. A feminine hygiene article (10) having a longitudinal centerline (L) and a transverse centerline (T) perpendicular to said longitudinal centerline (L), wherein said feminine hygiene article is not symmetric about said transverse centerline (T), said feminine hygiene article adapted to be worn in an undergarment (39) having a crotch portion (38) bounded on opposite sides by portions of curved leg openings (35), said feminine hygiene article comprising a main body portion, said main body portion comprising a liquid pervious body-facing surface (15), a liquid impervious garment facing surface (17), and an absorbent core (20) positioned between said body-facing surface and said garment-facing surface, said feminine hygiene article **characterized by**:
a. first and second flaps (15), said flaps extending laterally outwardly from longitudinal edges of said feminine hygiene article, each said flap being adapted to be folded over one of said portions of curved leg openings;
b. at least one indicator marker (34) visible from said body-facing surface, said indicator markers disposed to indicate proper alignment of said absorbent article with respect to said curved leg openings, said indicator marker having a ratio of length to width of at least about 1.5 and being oriented at an angle from the longiudinal axis of between about 10 degrees and about 30 degrees, wherein said indicator marker is printed marks.

4. A method for correctly placing a feminine hygiene article (10) in the crotch portion of an undergarment having a crotch portion bounded on opposite sides by portions ofcurved leg openings, the method **characterized by** the steps of:
a. providing a feminine hygiene article having at least one indicator marker (34) visible from said body-facing surface, said indicator marker disposed to indicate proper alignment of said feminine hygiene article with respect to said curved leg openings (35), wherein said at least one indicator marker is printed marks;
b. positioning said feminine hygiene article in said crotch portion (38) such that said at least one indicator marker is aligned with at least one leg elastic (36) of said curved leg openings; and
c. affixing said feminine hygiene article to said crotch portion of said undergarment.

5. The method according to Claim 4, wherein said method is performed while the undergarment is on the wearer by having the legs of the wearer being enclosed by respective leg elastics of said leg openings, the method further including the step of:
a. spreading said legs sufficiently to hold said crotch portion of said undergarment in a stretched condition such that said feminine hygiene article can be positioned in said positioning step (b).

## Patentansprüche

1. Damenhygieneartikel (10) mit einer Längsachse (L), der für das Tragen in einem Unterwäscheartikel (39) mit einem Schrittabschnitt (38), der auf beiden Seiten durch Abschnitte gekrümmter Beinöffnungen (35) begrenzt ist, konzipiert ist, wobei der Damenhygieneartikel einen Grundelementabschnitt umfasst, wobei der Grundelementabschnitt eine flüssigkeitsdurchlässige körperseitige Oberfläche (15), eine flüssigkeitsundurchlässige bekleidungsseitige Oberfläche (17) und einen Damenhygienekern (20), der zwischen der körperseitigen Oberfläche und der bekleidungsseitigen Oberfläche positioniert ist, umfasst, wobei der Damenhygieneartikel durch Folgendes gekennzeichnet ist:
a. mindestens eine Indikatormarkierung (34), die von der körperseitigen Oberfläche aus sichtbar ist, wobei die Indikatormarkierung so angeordnet ist, dass sie die sachgerechte Positionierung des Damenhygieneartikels (10) in Bezug auf die gekrümmten Beinöffnungen (35) anzeigt, wobei die mindestens eine Indikatormarkierung aus gedruckten Markierungen besteht.

2. Damenhygieneartikel aus Anspruch 1, wobei der Damenhygieneartikel eine Querachse (T) umfasst, die lotrecht zur Längsachse (L) verläuft, und wobei der Damenhygieneartikel im Bereich von mindestens einer von der Längs- (L) bzw. der Querachse (T) nicht symmetrisch ist.

3. Damenhygieneartikel (10) mit einer Längsachse (L) und einer Querachse (T) lotrecht zur Längsachse (L), wobei der Damenhygieneartikel im Bereich der Querachse (T) nicht symmetrisch ist, wobei der Damenhygieneartikel so konzipiert ist, dass er in einem Unterwäscheartikel (39) mit einem Schrittabschnitt (38), der auf beiden Seiten durch Abschnitte gekrümmter Beinöffnungen (35) begrenzt ist, getragen werden kann, wobei der Damenhygieneartikel einen Grundelementabschnitt umfasst, wobei der Grundelementabschnitt eine flüssigkeitsdurchlässige körperseitige Oberfläche (15), eine flüssigkeitsundurchlässige bekleidungsseitige Oberfläche (17) und einen Damenhygienekern (20), der zwischen der körperseitigen Oberfläche und der bekleidungsseitigen Oberfläche positioniert ist, umfasst, wobei der Damenhygieneartikel durch Folgendes gekennzeichnet ist:
a. erste und zweite Flügel (15), wobei sich die Flügel von den Längsrändern des Damenhygieneartikels seitlich nach außen erstrecken, wobei jeder Flügel so konzipiert ist, dass er über einen der Abschnitte der gekrümmten Beinöffnungen gefaltet werden kann;
b. mindestens eine Indikatormarkierung (34), die von der körperseitigen Oberfläche aus sichtbar ist, wobei die Indikatormarkierungen so angeordnet sind, dass sie die sachgerechte Positionierung des Absorptionsartikels in Bezug auf die gekrümmten Beinöffnungen anzeigen, wobei die Indikatormarkierung ein Länge-Breiten-Verhältnis von mindestens etwa 1,5 aufweist und von der Längsachse aus in einem Winkel von etwa 10 Grad bis etwa 30 Grad ausgerichtet ist, wobei die Indikatormarkierung aus gedruckten Markierungen besteht.

4. Verfahren für die sachgerechte Positionierung eines Damenhygieneartikels (10) im Schrittabschnitt eines Unterwäscheartikels mit einem Schrittabschnitt, der auf beiden Seiten durch Abschnitte gekrümmter Beinöffnungen begrenzt ist, wobei das Verfahren durch folgende Schritte gekennzeichnet ist:
a. Bereitstellen eines Damenhygieneartikels mit mindestens einer Indikatormarkierung (34), die von der körperseitigen Oberfläche aus sichtbar ist, wobei die Indikatormarkierung so angeordnet ist, dass sie die sachgerechte Positionierung des Damenhygieneartikels in Bezug auf die gekrümmten Beinöffnungen (35) anzeigt, wobei die mindestens eine Indikatormarkierung aus gedruckten Markierungen besteht;
b. Positionieren des Damenhygieneartikels im Schrittabschnitt (38) auf eine solche Weise, dass die mindestens eine Indikatormarkierung auf mindestens ein Beingummiband (36) der gekrümmten Beinöffnungen ausgerichtet ist; und
c. Befestigen des Damenhygieneartikels am Schrittabschnitt der Unterwäsche.

5. Verfahren nach Anspruch 4, wobei das Verfahren durchgeführt wird, während sich die Unterwäsche am Träger befindet, indem die Beine des Trägers durch die jeweiligen Beingummibänder der Beinöffnungen umschlossen werden, wobei das Verfahren ferner folgenden Schritt einschließt:
a. ausreichendes Spreizen der Beine, um den Schrittabschnitt der Unterwäsche in einem gedehnten Zustand zu halten, so dass der Damenhygieneartikel im Positionierungsschritt (b) positioniert werden kann.

## Revendications

1. Article d'hygiène féminine (10) ayant une ligne médiane longitudinale (L) et adapté pour être porté dans un sous-vêtement (39) ayant une partie d'entrejambe (38) délimitée sur des côtés opposés par des parties d'ouvertures de jambe courbées (35), ledit article d'hygiène féminine comprenant une partie de corps principale, ladite partie de corps principale comprenant une surface faisant face au corps perméable aux liquides (15), une surface faisant face au vêtement imperméable aux liquides (17), et une âme d'hygiène féminine (20) positionnée entre ladite surface faisant face au corps et ladite surface faisant face au vêtement, ledit article d'hygiène féminine **caractérisé par** :
a. au moins un marqueur indicateur (34) visible à partir de ladite surface faisant face au corps, ledit marqueur indicateur disposé pour indiquer un alignement correct dudit article d'hygiène féminine (10) par rapport auxdites ouvertures de jambe courbées (35), dans lequel ledit au moins un marqueur indicateur est constitué de marques imprimées.

2. Article d'hygiène féminine selon la revendication 1, où ledit article d'hygiène féminine comprend une ligne médiane transversale (T) perpendiculaire à ladite ligne médiane longitudinale (L), et où ledit article d'hygiène féminine n'est pas symétrique autour d'au moins l'une parmi ladite ligne médiane longitudinale (L) ou ladite ligne médiane transversale (T).

3. Article d'hygiène féminine (10) ayant une ligne médiane longitudinale (L) et une ligne médiane transversale (T) perpendiculaire à ladite ligne médiane longitudinale (L), où ledit article d'hygiène féminine n'est pas symétrique autour de ladite ligne médiane transversale (T), ledit article d'hygiène féminine adapté pour être porté dans un sous-vêtement (39) ayant une partie d'entrejambe (38) délimitée sur des côtés opposés par des parties d'ouvertures de jambe courbées (35), ledit article d'hygiène féminine comprenant une partie de corps principale, ladite partie de corps principale comprenant une surface faisant face au corps perméable aux liquides (15), une surface faisant face au vêtement imperméable aux liquides (17), et une âme d'hygiène féminine (20) positionnée entre ladite surface faisant face au corps et ladite surface faisant face au vêtement, ledit article d'hygiène féminine **caractérisé par** :
a. des premier et deuxième rabats (15), lesdits rabats s'étendant latéralement vers l'extérieur à partir des bords longitudinaux dudit article d'hygiène féminine, chaque rabat précité étant adapté pour être replié sur une desdites parties d'ouvertures de jambe courbées ;
b. au moins un marqueur indicateur (34) visible à partir de ladite surface faisant face au corps, lesdits marqueurs indicateurs disposés pour indiquer un alignement correct dudit article absorbant par rapport auxdites ouvertures de jambe courbées, ledit marqueur indicateur ayant un rapport de longueur sur largeur d'au moins environ 1,5 et étant orienté selon un angle par rapport à l'axe longitudinal compris entre environ 10 degrés et environ 30 degrés, dans lequel ledit marqueur indicateur est constitué de marques imprimées.

4. Procédé de mise en place correcte d'un article d'hygiène féminine (10) dans la partie d'entrejambe d'un sous-vêtement ayant une partie d'entrejambe délimitée sur des côtés opposés par des parties d'ouvertures de jambe courbées, le procédé **caractérisé par** les étapes consistant à :
a. fournir un article d'hygiène féminine ayant au moins un marqueur indicateur (34) visible à partir de ladite surface faisant face au corps, ledit marqueur indicateur disposé pour indiquer un alignement correct dudit article d'hygiène féminine par rapport auxdites ouvertures de jambe courbées (35), dans lequel ledit au moins un marqueur indicateur est constitué de marques imprimées ;
b. positionner ledit article d'hygiène féminine dans ladite partie d'entrejambe (38) de telle sorte que ledit au moins un marqueur indicateur est aligné avec au moins un élastique de jambe (36) desdites ouvertures de jambe courbées ; et,
c. fixer ledit article d'hygiène féminine à ladite partie d'entrejambe dudit sous-vêtement.

5. Procédé selon la revendication 4, où ledit procédé est effectué alors que le sous-vêtement est sur le porteur en ayant les jambes du porteur ceinturées par les élastiques de jambe respectifs desdites ouvertures de jambe, le procédé incluant en outre l'étape consistant à :
a. écarter suffisamment lesdites jambes pour maintenir ladite partie d'entrejambe dudit sous-vêtement dans une condition étirée de telle sorte que ledit article d'hygiène féminine peut être positionné dans ladite étape de positionnement (b).
